(19) Europäisches Patentamt
European Patent Office
Office européen des brevets

(11) EP 3 047 850 A1

(12) EUROPEAN PATENT APPLICATION
published in accordance with Art. 153(4) EPC

(43) Date of publication:
27.07.2016 Bulletin 2016/30

(21) Application number: 14845745.0

(22) Date of filing: 18.09.2014

(51) Int Cl.:
*A61K 31/444* (2006.01) *A61K 9/08* (2006.01)
*A61K 47/34* (2006.01) *A61P 3/10* (2006.01)
*A61P 27/02* (2006.01) *A61P 27/06* (2006.01)
*A61P 35/00* (2006.01)

(86) International application number:
PCT/JP2014/074698

(87) International publication number:
WO 2015/041294 (26.03.2015 Gazette 2015/12)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 20.09.2013 JP 2013195329

(71) Applicant: Santen Pharmaceutical Co., Ltd
Osaka-shi
Osaka 533-8651 (JP)

(72) Inventors:
- MURAI, Kenji
Ikoma-shi
Nara 630-0101 (JP)
- YAMADA, Kazuhito
Ikoma-shi
Nara 630-0101 (JP)

(74) Representative: Müller-Boré & Partner
Patentanwälte PartG mbB
Friedenheimer Brücke 21
80639 München (DE)

# (54) POLYETHYLENE GLYCOL-CONTAINING COMPOSITION

(57) The present invention relates to a pharmaceutical composition comprising a compound represented by the formula (1) or a salt thereof and polyethylene glycol.

EP 3 047 850 A1

## Description

TECHNICAL FIELD

The present invention relates to a pharmaceutical composition comprising a compound represented by the formula (1):

[Formula 1]

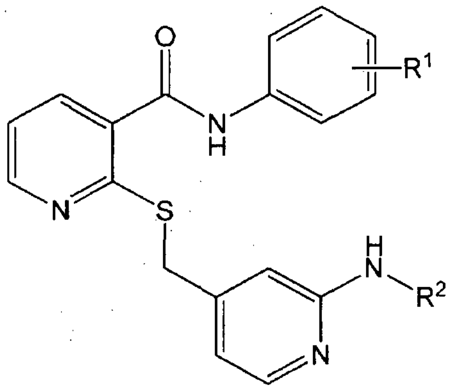

(1)

[wherein

$R^1$ represents a hydrogen atom, a halogen atom, a hydroxyl group, a $C_{1-6}$ alkyl group, a $C_{1-6}$ alkyl group substituted by one or more halogen atoms, a $C_{1-6}$ alkoxyl group or a $C_{1-6}$ alkoxyl group substituted by one or more halogen atoms; and
$R^2$ represents a hydrogen atom, a $C_{1-6}$ alkyl group, a $C_{1-6}$ alkylcarbonyl group or a $C_{1-6}$ alkylcarbonyl group substituted by one or more hydroxyl groups]

or a salt thereof and polyethylene glycol, and a method for stabilizing said compound or a salt thereof.

BACKGROUND ART

It has been disclosed in Patent Document 1 that the compound represented by the formula (1) shows a cell proliferation inhibiting action in the test system using a VEGF-induced HUVEC proliferation reaction evaluating system, shows a tumor proliferation inhibiting action in the test system using a tumor-bearing mouse model, shows a feet swollen inhibiting action in the test system using a rat adjuvant arthritis model, and shows a choroidal neovascular inhibiting action in the test system using a rat choroidal neovascularization model. Further, the compound represented by the formula (1) is useful as a medicine from their pharmacological function, in particular, it has been disclosed that it is expected to be a prophylaxis or treatment agent of the diseases such as cancer, rheumatoid arthritis, age-related macular degeneration, diabetic retinopathy and diabetic macular edema, etc.

Also, in Patent Document 2, there are disclosed a benzenesulfonic acid salt of 2-[ [[2-[(hydroxyacetyl)amino]-4-pyridinyl]methyl]thio]-N-[4-(trifluoromethoxy)-phenyl]-3-pyridinecaboxamide, which is one of the compound represented by the formula (1), crystal thereof, crystal polymorphism thereof and processes for producing these. In addition, it has also been disclosed that a benzenesulfonic acid salt of 2-[[[2-[(hydroxyacetyl)amino] -4-pyridinyl]methyl] thio] -N-[4-(trifluoromethoxy)phenyl] -3-pyridinecaboxamide is excellent in storage stability, and no deposition of minerals is recognized in the stomach even when repeated oral administration is carried out.

On the other hand, in Patent Documents 3 to 5, there are disclosed a composition for ophthalmology containing N-[4-(3-amino-1H-indazol-4-yl)phenyl]-N'-(2-fluoro-5-methylphenyl)urea which is a compound inhibiting receptor tyrosine kinase and polyethylene glycol.

Further, in Patent Document 6, a composition for ophthalmology containing rapamycin and polyethylene glycol has been disclosed.

However, in Patent Documents 1 to 6, a pharmaceutical composition containing a compound represented by the formula (1) or a salt thereof and polyethylene glycol is not disclosed, and it is never disclosed that the polyethylene glycol improves stability of the pharmacologically active compound in the pharmaceutical composition.

PRIOR ART DOCUMENTS

PATENT DOCUMENTS

Patent Document 1: US Patent Application Publication No. 2007/0149574A
Patent Document 2: US Patent Application Publication No. 2012/0116088A
Patent Document 3: WO 2007/076358A
Patent Document 4: WO2009/014510A
Patent Document 5: WO2010/101971A
Patent Document 6: US Patent No. 8,367,097

SUMMARY OF INVENTION

TECHNICAL PROBLEM

The present inventors have found that, in the development stage of a pharmaceutical composition containing the compound represented by the above-mentioned formula (1) or a salt thereof (in the following, it is also referred to as "the present compound"), stability of the present compound is remarkably lowered in a pharmaceutical composition in which the present compound is being dissolved.

An object of the present invention is to provide a pharmaceutical composition in which the pharmaceutical composition contains the present compound, the present compound in the pharmaceutical composition is stable, and the pharmaceutical composition sustains release of the present compound.

SOLUTION TO PROBLEM

The present inventors have intensively studied about the solvent (for example, polyethylene glycol, dimethylsulfoxide, N-methylpyrrolidone, N,N-dimethylacetamide) to dissolve the present compound to solve the above-mentioned problems, and as a result, they have found that the present compound in a pharmaceutical composition has high residual ratio under long term storage wherein polyethylene glycol is used for. The present invention has been accomplished.

That is, the present invention relates to the following.

(1) A pharmaceutical composition comprising a compound represented by the above-mentioned formula (1) or a salt thereof and polyethylene glycol.

(2) The pharmaceutical composition described in the above-mentioned (1), wherein, in the above-mentioned formula (1),

$R^1$ represents a $C_{1-6}$ alkoxyl group or a $C_{1-6}$ alkoxyl group substituted by one or more halogen atoms; and
$R^2$ represents a $C_{1-6}$ alkylcarbonyl group or a $C_{1-6}$ alkylcarbonyl group substituted by one or more hydroxyl groups.

(3) The pharmaceutical composition described in the above-mentioned (1), wherein, in the above-mentioned formula (1),
$R^1$ represents a $C_{1-6}$ alkoxyl group substituted by one or more halogen atoms; and
$R^2$ represents a $C_{1-6}$ alkylcarbonyl group substituted by one or more hydroxyl groups.

(4) The pharmaceutical composition described in the above-mentioned (1), wherein the compound represented by the above-mentioned formula (1) is 2-[[[2-[(hydroxyacetyl)-amino]-4-pyridinyl]methyl]thio]-N-[4-(trifluoromethoxy)phenyl]-3-pyridine-caboxamide.

(5) The pharmaceutical composition described in the above-mentioned any one of (1) to (4), wherein an average molecular weight of the polyethylene glycol is within the range of 100 to 2,000.

(6) The pharmaceutical composition described in the above-mentioned any one of (1) to (4), wherein an average molecular weight of the polyethylene glycol is within the range of 200 to 600.

(7) The pharmaceutical composition described in the above-mentioned any one of (1) to (4), wherein the polyethylene glycol is PEG 400.

(8) The pharmaceutical composition described in the above-mentioned any one of (1) to (7), wherein the amount of the polyethylene glycol in the pharmaceutical composition is 70 to 99.99% (w/w).

(9) The pharmaceutical composition described in the above-mentioned any one of (1) to (8), wherein the amount

of the compound represented by the above-mentioned formula (1) or a salt thereof is 0.01 to 20% (w/v).

(10) The pharmaceutical composition described in the above-mentioned any one of (1) to (9), wherein it is used for prophylaxis or treatment of an eye disease.

(11) The pharmaceutical composition described in the above-mentioned (10), wherein the eye disease is age-related macular degeneration, diabetic retinopathy, retinopathy of prematurity, retinal vein occlusion, retinal artery occlusion, polypoidal choroidal vasculopathy, retinal angiomatous proliferation, myopic choroidal neovascularization, diabetic macular edema, ocular tumor, radiation retinopathy, iris rubeosis, neovascular glaucoma or proliferative vitreoretinopathy (PVR).

(12) The pharmaceutical composition described in the above-mentioned (10) or (11), wherein it is for intravitreal administration.

(13) The pharmaceutical composition described in the above-mentioned (12), wherein it is administered with 1 to 100 μL per one time.

(14) The pharmaceutical composition described in the above-mentioned (12) or (13), wherein it is administered with an interval of once a week to once 3 years.

(15) A method for stabilizing the compound represented by the above-mentioned formula (1) or a salt thereof which comprises dissolving the above-mentioned formula (1) or a salt thereof in polyethylene glycol.

(16) The method described in the above-mentioned (15), wherein the compound represented by the above-mentioned formula (1) is 2-[[[2-[(hydroxyacetyl)amino]-4-pyridinyl]methyl]thio]-N-[4-(trifluoromethoxy)phenyl]-3-pyridinecaboxamide.

Further, the present invention relates to the following.

(17) The method described in the above-mentioned (15), wherein, in the above-mentioned formula (1),

$R^1$ represents a $C_{1-6}$ alkoxyl group or a $C_{1-6}$ alkoxyl group substituted by one or more halogen atoms; and $R^2$ represents a $C_{1-6}$ alkylcarbonyl group or a $C_{1-6}$ alkylcarbonyl group substituted by one or more hydroxyl groups. (18) The pharmaceutical composition described in the above-mentioned (15), wherein, in the above-mentioned formula (1),

$R^1$ represents a $C_{1-6}$ alkoxyl group substituted by one or more halogen atoms; and

$R^2$ represents a $C_{1-6}$ alkylcarbonyl group substituted by one or more hydroxyl groups.

(19) The pharmaceutical composition described in the above-mentioned any one of (1) to (16), wherein it is for a long term preservation.

(20) The pharmaceutical composition described in the above-mentioned any one of (1) to (16), wherein it is for sustained release.

Incidentally, each of the constitutions of the above-mentioned (1) to (20) may be combined by optionally selecting 2 or more.

## ADVANTAGEOUS EFFECTS OF INVENTION

According to the present invention, a pharmaceutical composition can be provided in which the present compound in the pharmaceutical composition has been stabilized for a long term. In addition, the pharmaceutical composition of the present invention sustains release of the present compound, and effective against the retinochoroidal vascular permeability promotion model for a long period of time, so that it is useful as a prophylaxis or treatment agent for age-related macular degeneration, diabetic retinopathy, retinopathy of prematurity, retinal vein occlusion, retinal artery occlusion, polypoidal choroidal vasculopathy, retinal angiomatous proliferation, myopic choroidal neovascularization, diabetic macular edema, ocular tumor, radiation retinopathy, iris rubeosis, neovascular glaucoma, proliferative vitreoretinopathy (PVR), etc. Further, the pharmaceutical composition of the present invention has sufficient safety as a medicine.

## DESCRIPTION OF EMBODIMENTS

In the following, the present invention is explained in detail.

The pharmaceutical composition of the present invention contains the compound represented by the above-mentioned formula (1) or a salt thereof (the present compound).

The "halogen atom" means fluorine, chlorine, bromine or iodine.

The "$C_{1-6}$ alkyl group" represents a linear or branched alkyl group having 1 to 6 carbon atoms, and preferably a linear or branched alkyl group having 1 to 4 carbon atoms. Specific examples include a methyl group, an ethyl group,

an n-propyl group, an n-butyl group, an n-pentyl group, an n-hexyl group, an isopropyl group, an isobutyl group, a sec-butyl group, a tert-butyl group, an isopentyl group, etc.

The "$C_{1-6}$ alkoxyl group" represents a group in which the hydrogen atom(s) of the hydroxyl group(s) is/are substituted by the above-mentioned $C_{1-6}$ alkyl group. Specific examples include a methoxy group, an ethoxy group, an n-propoxy group, an n-butoxy group, an n-pentoxy group, an n-hexyloxy group, an isopropoxy group, an isobutoxy group, a sec-butoxy group, a tert-butoxy group, an isopentyloxy group, etc.

The "$C_{1-6}$ alkylcarbonyl group" represents a group in which the hydrogen atom of the formyl group is substituted by the above-mentioned $C_{1-6}$ alkyl group. Specific examples include a methylcarbonyl group (an acetyl group), an ethylcarbonyl group, an n-propylcarbonyl group, an n-butylcarbonyl group, an n-pentylcarbonyl group, an n-hexylcarbonyl group, an isopropylcarbonyl group, an isobutylcarbonyl group, a secbutylcarbonyl group, a tert-butylcarbonyl group, an isopentylcarbonyl group, etc.

The terms "substituted by one or more halogen atoms" referred to in the present invention" means that the above-mentioned $C_{1-6}$ alkyl group is substituted by one or more and a substitutable number or less of the halogen atoms. The respective halogen atoms may be the same or different from each other, and a number of the halogen atoms is preferably the case of 2 or 3, particularly preferably the case of 3.

The terms "substituted by one or more hydroxyl groups" referred to in the present invention" means that the above-mentioned $C_{1-6}$ alkyl group is substituted by one or more and a substitutable number or less of the hydroxyl groups. A number of the hydroxyl groups is preferably the case of 1 or 2, particularly preferably the case of 1.

Also, the present compound in the present invention contains a derivative such as an ester, an amide, etc. Specific examples of the ester may be exemplified by an ester in which the hydroxyl group(s) in the present compound and a carboxylic acid such as acetic acid, propionic acid, isopropionic acid, butyric acid, isobutyric acid, pivalic acid, etc., are condensed. Specific examples of the amide may be exemplified by an amide in which the amino group in the present compound and a carboxylic acid such as acetic acid, propionic acid, isopropionic acid, butyric acid, isobutyric acid, pivalic acid, etc., are condensed.

In addition, the present compound may have a form of a hydrate or a solvate.

When geometric isomer, tautomer or optical isomer is present in the present compound, these isomers are also included in the scope of the present invention.

Further, when crystal polymorphism is present in the present compound, crystal polymorphs are also included in the scope of the present invention.

Preferred examples of (a) the compound represented by the formula (1) include a compound in which the respective groups in the formula (1) are the groups shown below, or a salt thereof.

(a1) $R^1$ represents a $C_{1-6}$ alkoxyl group or a $C_{1-6}$ alkoxyl group substituted by one or more halogen atoms; and/or
(a2) $R^2$ represents a $C_{1-6}$ alkylcarbonyl group or a $C_{1-6}$ alkylcarbonyl group substituted by one or more hydroxyl groups.

That is, the compound represented by the formula (1), a compound or a salt thereof comprising one or two or more respective combinations selected from the above-mentioned (a1) and (a2) include as a preferred example(s).

(b) More preferred example of the compound represented by the formula (1) includes a compound or a salt thereof in which each group in the formula (1) is a group mentioned below.
(b1) $R^1$ represents a $C_{1-6}$ alkoxyl group substituted by one or more halogen atoms; and/or
(b2) $R^2$ represents a $C_{1-6}$ alkylcarbonyl group substituted by one or more hydroxyl groups.

That is, the compound represented by the formula (1) includes a compound comprising 1 or 2 or more combinations selected from the above-mentioned (b1) and (b2) or a salt thereof as a preferred example. Also, the selected conditions may be combined with the conditions of (a).

Most preferred example of (c) the compound represented by the formula (1) is the compound (2-[[[2-[(hydroxyacetyl)amino]-4-pyridinyl]methyl]-thio]-N-[4-(trifluoromethoxy)phenyl]-3-pyridinecaboxamide) represented by the formula (2):

[Formula 2]

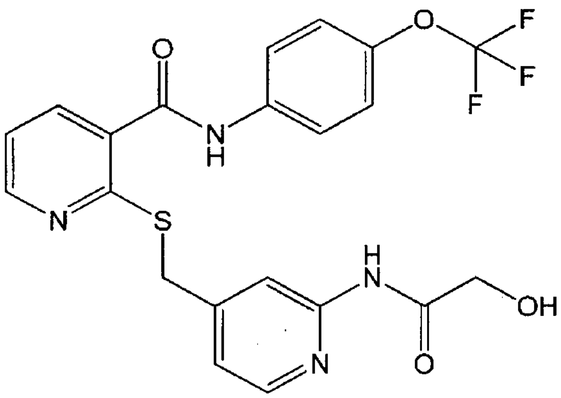

(2)

or a salt thereof.

The compound represented by the formula (1) or a salt thereof contained in the pharmaceutical composition of the present invention can be manufactured according to the usual method in this field of the art such as the method disclosed in the specification of US Patent Application Publication No. 2007/0149574, etc.

In the pharmaceutical composition of the present invention, in stead of the compound represented by the formula (1), the effect of the present invention can be obtained when a tyrosine kinase inhibitor selected from the group consisting of Tafetinib, SIM-817378, ACTB-1003, Chiauranib, CT-53608, Cinnamon, chim4G8-SDIE, CEP-5214, IMC-1C11, CEP-7055, 3-[5-[2-[N-(2-Methoxyethyl)-N-methylamino]ethoxy]-1H-indol-2-yl]quinolin-2(1H)-one, hF4-3C5, ZK-CDK, IMC-EB10, LS-104, CYC-116, OSI-930, PF-337210, JNJ-26483327, SSR-106462, R-1530, PRS-050, TG-02, SC-71710, SB-1578, AMG-191, AMG-820, Sulfatinib, Lucitanib hydrochloride, JNJ-28312141, Ilorasertib, PLX-5622, ARRY-382, TAS-115, Tanibirumab, Henatinib, LY-2457546, PLX-7486, FPA-008, NVP-AEE-788, cgi-1842, RAF-265, MK-2461, SG-00529, Rebastinib, Golvatinib, Roniciclib, BVT-II, X-82, XV-615, KD-020, Lestaurtinib, Delphinidin, Semaxanib, Vatalanib, OSI-632, Telatinib, Alacizumab pegol, ATN-224, Tivozanib, XL-999, Icrucumab, Foretinib, Crenolanib besylate, R-406, Brivanib, Pegdinetanib, TG-100572, Olaratumab, Fostamatinib disodium, BMS-690514, AT-9283, MGCD-265, Quizartinib, ENMD-981693, Famitinib, Anlotinib, Tovetumab, PLX-3397, Fruquintinib, (-)-Epigallocatechin, Midostaurin, NSC-706456, Orantinib, Cediranib, Dovitinib, XL-647, Motesanib, Linifanib, Brivanib, Cediranib, Apatinib, Fedratinib, Pacritinib, Ramucirumab, Intedanib, Masitinib, Elemene, Dihydroartemisinin, WS-1442, Itraconazole, Leflunomide, Dihydroartemisinin, Imatinib, Sorafenib, Sunitinib, Dasatinib, Pazopanib, Vandetanib, Axitinib, Regorafenib, Cabozantinib and Ponatinib is used. That is, it is possible to provide a pharmaceutical composition in which these tyrosine kinase inhibitors in the pharmaceutical composition are stabilized for a long period of time. Moreover, the pharmaceutical composition sustaines release of the tyrosine kinase inhibitor, is effective against a retinochoroidal vascular permeability promotion model for a long period of time, and useful as a prophylaxis or treatment agent for age-related macular degeneration, diabetic retinopathy, retinopathy of prematurity, retinal vein occlusion, retinal artery occlusion, polypoidal choroidal vasculopathy, retinal angiomatous proliferation, myopic choroidal neovascularization, diabetic macular edema, ocular tumor, radiation retinopathy, iris rubeosis, neovascular glaucoma, proliferative vitreoretinopathy (PVR), etc. Further, the pharmaceutical composition has sufficient safety as a medicine.

In the pharmaceutical composition of the present invention, a salt of the compound represented by the formula (1) is not particularly limited so long as it is acceptable as a pharmaceutical, and the salt includes a salt with an inorganic acid, a salt with an organic acid, a quaternary ammonium salt, a salt with a halogen ion, a salt with an alkali metal, a salt with an alkaline earth metal, a metal salt, a salt with an organic amine, etc. The salt with the inorganic acid includes a salt with hydrochloric acid, hydrobromic acid, hydroiodic acid, nitric acid, sulfuric acid, phosphoric acid, etc. The salt with the organic acid includes a salt with acetic acid, oxalic acid, fumaric acid, maleic acid, succinic acid, malic acid, citric acid, tartaric acid, adipic acid, gluconic acid, glucoheptonic acid, glucuronic acid, terephthalic acid, methanesulfonic acid, alanine, lactic acid, hippuric acid, 1,2-ethanedisulfonic acid, isethionic acid, lactobionic acid, oleic acid, gallic acid, pamoic acid, polygalacturonic acid, stearic acid, tannic acid, trifluoromethanesulfonic acid, benzenesulfonic acid, p-toluenesulfonic acid, lauryl sulfate, methyl sulfate, naphthalenesulfonic acid, sulfosalicylic acid, etc. The quaternary ammonium salt includes a salt with methyl bromide, methyl iodide, etc. The salt with the halogen ion includes a salt with a chloride ion, a bromide ion, an iodide ion, etc., the salt with an alkali metal includes a salt with lithium, sodium, potassium, etc., the salt with an alkaline earth metal includes a salt with calcium, magnesium, etc., the metal salt includes a salt with iron, zinc, etc. The salt with the organic amine includes a salt with triethylenediamine, 2-aminoethanol, 2,2-iminobis(ethanol), 1-deoxy-1-(methylamino)-2-D-sorbitol, 2-amino-2-(hydroxymethyl)-1,3-propanediol, procaine, N,N-bis(phenylmethyl)-1,2-ethanediamine, etc.

In the pharmaceutical composition of the present invention, a concentration of the compound represented by the formula (1) or a salt thereof is not particularly limited so long as it is an amount sufficient for exhibiting a desired medicinal effect, and preferably 0.01 to 20% (w/v), more preferably 0.1 to 15% (w/v), further preferably 0.5 to 12% (w/v), further more preferably 1 to 10% (w/v), particularly preferably 1 to 8% (w/v), and most preferably 1% (w/v), 1.5% (w/v), 2% (w/v), 2.5% (w/v), 3% (w/v), 3.5% (w/v), 4% (w/v), 5% (w/v), 6% (w/v), 7% (w/v) or 8% (w/v).

The polyethylene glycol (PEG) contained in the pharmaceutical composition of the present invention is a polyether in which ethylene glycols are polymerized, which is represented by the chemical formula $HO(CH_2CH_2O)_nH$, where n is a number of polymerization. The polyethylene glycol (PEG) may be used those commercially available, or those produced according to the usual method in this field of the art.

In the pharmaceutical composition of the present invention, an average molecular weight of the polyethylene glycol is preferably 100 to 2,000, more preferably 100 to 1,000, further preferably 100 to 800, more preferably 200 to 600, further more preferably 400 to 600, particularly preferably 400 and 600, and most preferably 400. Specific examples of the polyethylene glycol include PEG 100, PEG 200, PEG 300, PEG 400, PEG 600, PEG 800, etc.

In the pharmaceutical composition of the present invention, the amount of the polyethylene glycol is preferably 70 to 99.99% (w/w), more preferably 80 to 99.9% (w/w), further preferably 90 to 99.5% (w/w), particularly preferably 92 to 99.3% (w/w), and most preferably 93 to 99% (w/w).

In the pharmaceutical composition of the present invention, an additive may be used, if necessary, and as the additive, a surfactant, a buffer, an isotonicifier, a stabilizer, an anticeptic, an antioxidant, a high-molecular weight polymer, etc., may be added.

In the pharmaceutical composition of the present invention, a surfactant which can be used as an additive(s) for the medicine, for example, a cationic surfactant, an anionic surfactant and/or a nonionic surfactant may be formulated. Examples of the anionic surfactant include phospholipid, etc., and the phospholipid include lecithin, etc. Examples of the cationic surfactant include an alkylamine salt, an alkylamine polyoxyethylene adduct, a fatty acid triethanolamine monoester salt, an acylaminoethyldiethylamine salt, a fatty acid polyamine condensate, an alkyltrimethyl ammonium salt, a dialkyldimethyl ammonium salt, an alkyldimethyl-benzyl ammonium salt, an alkyl pyridinium salt, an acylaminoalkyl type ammonium salt, an acylaminoalkyl pyridinium salt, a diacyloxyethyl ammonium salt, an alkylimidazoline, a 1-acylaminoethyl-2-alkylimidazoline, a 1-hydroxylethyl-2-alkylimidazoline, etc. The alkyldimethylbenzyl ammonium salt include benzalkonium chloride, cetalkonium chloride, etc. Examples of the nonionic surfactant include a polyoxyethylene fatty acid ester, a polyoxyethylene sorbitan fatty acid ester, a polyoxyethylene hardened castor oil, a polyoxyethylene castor oil, a polyoxyethylene polyoxypropylene glycol, a sucrose fatty acid ester, vitamin E TPGS, etc.

The polyoxyethylene fatty acid ester include polyoxyl 40 stearate, etc.

The polyoxyethylene sorbitan fatty acid ester include polysorbate 80, polysorbate 60, polysorbate 40, polyoxyethylene sorbitan monolaurate, polyoxyethylene sorbitan trioleate, polysorbate 65, etc.

As the polyoxyethylene hardened castor oil, various polyoxyethylene hardened castor oils different in the polymerization number of the ethylene oxide may be used, and the polymerization number of the ethylene oxide is preferably 10 to 100, more preferably 20 to 80, particularly preferably 40 to 70, and most preferably 60. Specific examples of the polyoxyethylene hardened castor oil include polyoxy-ethylene hardened castor oil 10, polyoxyethylene hardened castor oil 40, polyoxy-ethylene hardened castor oil 50, polyoxyethylene hardened castor oil 60, etc.

As the polyoxyethylene castor oil, various polyoxyethylene castor oils different in the polymerization number of the ethylene oxide may be used, and the polymerization number of the ethylene oxide is preferably 5 to 100, more preferably 20 to 50, particularly preferably 30 to 40, and most preferably 35. Specific examples of the polyoxyethylene castor oil include Polyoxyl 5 castor oil, Polyoxyl 9 castor oil, Polyoxyl 15 castor oil, Polyoxyl 35 castor oil, Polyoxyl 40 castor oil, etc.

The polyoxyethylene polyoxypropylene glycol include polyoxyethylene (160) polyoxypropylene (30) glycol, polyoxyethylene (42) polyoxypropylene (67) glycol, polyoxyethylene (54) polyoxypropylene (39) glycol, polyoxyethylene (196) polyoxypropylene (67) glycol, polyoxyethylene (20) polyoxypropylene (20) glycol, etc.

The sucrose fatty acid ester include polyoxyl 40 stearate, etc.

The vitamin E TPGS is also called as tocopherol polyethylene glycol 1000 succinate.

Into the pharmaceutical composition of the present invention, a buffer which can be used as an additive for a medicine may be formulated. Examples of the buffer include phosphoric acid or a salt thereof, boric acid or a salt thereof, citric acid or a salt thereof, acetic acid or a salt thereof, carbonic acid or a salt thereof, tartaric acid or a salt thereof, ε-aminocaproic acid, trometamol, etc. Examples of the phosphate include sodium phosphate, sodium dihydrogen phosphate, disodium hydrogen phosphate, potassium phosphate, potassium dihydrogen phosphate, dipotassium hydrogen phosphate, etc., the borate include borax, sodium borate, potassium borate, etc., the citrate include sodium citrate, disodium citrate, etc., the acetate include sodium acetate, potassium acetate, etc., the carbonate include sodium carbonate, sodium hydrogen carbonate, etc., and the tartarate include sodium tartarate, potassium tartarate, etc.

Into the pharmaceutical composition of the present invention, an isotonicifier which can be used as an additive for a medicine may be optionally formulated. Examples of the isotonicifier include an ionic isotonicifier and a nonionic

isotonicifier, etc. The ionic isotonicifier include sodium chloride, potassium chloride, calcium chloride, magnesium chloride, etc., and the nonionic isotonicifier include glycerin, propylene glycol, sorbitol, mannitol, etc.

Into the pharmaceutical composition of the present invention, a stabilizer which can be used as an additive for a medicine may be optionally formulated. Examples of the stabilizer include edetic acid, sodium edetate, sodium citrate, etc.

Into the pharmaceutical composition of the present invention, an anticeptic which can be used as an additive for a medicine may be optionally formulated. Examples of the anticeptic include benzalkonium chloride, benzalkonium bromide, benzethonium chloride, sorbic acid, potassium sorbate, methyl paraoxybenzoate, propyl paraoxybenzoate, chlorobutanol, etc.

Into the pharmaceutical composition of the present invention, an antioxidant which can be used as an additive for a medicine may be optionally formulated. Examples of the antioxidant include ascorbic acid, tocopherol, dibutylhydroxytoluene, butylhydroxyanisole, sodium erythorbate, propyl gallate, sodium sulfite, etc.

Into the pharmaceutical composition of the present invention, a high-molecular weight polymer which can be used as an additive for a medicine may be optionally formulated. Examples of the high-molecular weight polymer include methyl cellulose, ethyl cellulose, hydroxymethyl cellulose, hydroxyethyl cellulose, hydroxypropyl cellulose, hydroxyethyl methyl cellulose, hydroxypropyl methyl cellulose, carboxymethyl cellulose, sodium carboxymethyl cellulose, hydroxypropyl methyl cellulose acetate succinate, hydroxypropyl methyl cellulose phthalate, carboxymethyl ethyl cellulose, cellulose phthalate acetate, polyvinylpyrrolidone, polyvinyl alcohol, carboxyvinyl polymer, polyethylene glycol, etc.

A concentration of the additives when the additive(s) is/are formulated into the pharmaceutical composition of the present invention may be optionally adjusted depending on the kind(s) of the additive(s), and the total amount thereof is preferably 0.0001 to 20% (w/v), more preferably 0.001 to 10% (w/v), further preferably 0.01 to 8% (w/v), particularly preferably 0.1 to 5% (w/v), and most preferably 1 to 3% (w/v).

Into the pharmaceutical composition of the present invention, a solvent which can be used as an additive for a medicine may be optionally formulated. Examples of the solvent include dimethylsulfoxide, N-methylpyrrolidone, N,N-dimethylacetamide, ethanol, etc.

A concentration of the additives when the solvent(s) is/are formulated into the pharmaceutical composition of the present invention may be optionally adjusted depending on the kind(s) of the solvent(s), and the total amount thereof is preferably 0.1 to 30% (w/v), more preferably 1 to 20% (w/v), further preferably 1.5 to 15% (w/v), particularly preferably 2 to 10% (w/v), and most preferably 3 to 7% (w/v).

In the pharmaceutical composition of the present invention, a pharmaceutical composition substantially containing the compound represented by the formula (1) or a salt thereof and polyethylene glycol alone is preferred. In this case, a concentration of the compound represented by the formula (1) or a salt thereof is preferably 0.01 to 20% (w/v), more preferably 0.1 to 15% (w/v), further preferably 0.5 to 12% (w/v), further more preferably 1 to 10% (w/v), particularly preferably 1 to 8% (w/v), and most preferably 1% (w/v), 1.5% (w/v), 2% (w/v), 2.5% (w/v), 3% (w/v), 3.5% (w/v), 4% (w/v), 5% (w/v), 6% (w/v), 7% (w/v) or 8% (w/v).

The pharmaceutical composition of the present invention may be administered orally or parenterally. The dosage form of the pharmaceutical composition of the present invention is not particularly limited so long as it can be used as a medicine. The dosage form include, for example, as the oral agent, a liquid agent, a suspension, a tablet, a capsule, a granule and a powder, and as the parenteral agent, include an injection agent, an infusion, a nasal drop, an ear drop, an eye drop, etc. It is preferably mentioned an injection for ophthalmology and an eye drop, more preferably mentioned an injection for ophthalmology, and most preferably mentioned an injection for intravitreal administration. These can be manufactured according to the usual manner in this field of the art.

The pharmaceutical composition of the present invention can be optionally administered depending on the dosage form. In the case of the injection for ophthalmology, for example, it can be administered into the vitreous body, at the neighbor of posterior sclera, at surrounding the eye socket, or between the sclera and the conjunctiva. When the injection for ophthalmology is, for example, administered into the vitreous body, the administration dose is not particularly limited so long as it is an amount sufficient for exhibiting a desired medicinal effect, and is, per one time, preferably 1 to 100 $\mu$L, more preferably 5 to 50 $\mu$L, further preferably 10 to 30 $\mu$L, and most preferably 10 $\mu$L, 20 $\mu$L or 30 $\mu$L. An administration dose of the present compound is preferably 0.001 to 30 mg/eye, more preferably 0.01 to 10 mg/eye, further preferably 0.1 to 5 mg/eye, particularly preferably 0.2 to 1.6 mg/eye, and most preferably 0.2 mg/eye, 0.3 mg/eye, 0.4 mg/eye, 0.5 mg/eye, 0.6 mg/eye, 0.7 mg/eye, 0.8 mg/eye, 1 mg/eye, 1.2 mg/eye, 1.4 mg/eye or 1.6 mg/eye.

When the pharmaceutical composition of the present invention is continuously administered into the vitreous body, the administration interval is not particularly limited so long as it is an amount sufficient for exhibiting a desired medicinal effect, and preferably administered with an interval of once a week to once 3 years, more preferably administered with an interval of once a week, once 2 weeks, once a month, once 2 months, once 3 months, once 4 months, once 5 months, once 6 months, once a year, once 2 years or once 3 years, and most preferably administered with an interval of once 2 months, once 3 months, once 4 months, once 5 months or once 6 months. In addition, the administration interval may be optionally changed.

The composition of the present invention is useful as a medicine, and can be used as a prophylaxis or treatment agent for age-related macular degeneration, diabetic retinopathy, retinopathy of prematurity, retinal vein occlusion, retinal artery occlusion, polypoidal choroidal vasculopathy, retinal angiomatous proliferation, myopic choroidal neovascularization, diabetic macular edema, ocular tumor, radiation retinopathy, iris rubeosis, neovascular glaucoma, proliferative vitreoretinopathy (PVR), etc.

In the following, preparation examples and test results are shown, and these are to better understand the present invention, which are not intended to limit the scope of the present invention.

Preparation examples

In the following, representative preparation examples using the present compound are shown. Incidentally, in the following mentioned preparation examples, a formulation amount of each component is a content in 100 mL of the composition.

Preparation example 1

| | |
|---|---|
| Present compound | 0.01 to 20 g |
| PEG 400 | Quantum sufficit |

Preparation example 2

| | |
|---|---|
| Present compound | 0.01 to 20 g |
| Dimethylsulfoxide | 0.1 to 30 g |
| PEG 400 | Quantum sufficit |

Preparation example 3

| | |
|---|---|
| Present compound | 0.01 to 20 g |
| Polysorbate 20 | 0.0001 to 20 g |
| PEG 400 | Quantum sufficit |

Preparation example 4

| | |
|---|---|
| Present compound | 0.01 to 20 g |
| Polyoxyethylene hardened castor oil 60 | 0.0001 to 20 g |
| PEG 400 | Quantum sufficit |

Preparation example 5

| | |
|---|---|
| Present compound | 0.01 to 20 g |
| Polyoxyl 35 castor oil | 0.0001 to 20 g |
| PEG 400 | Quantum sufficit |

Incidentally, a desired composition can be obtained by optionally adjusting a kind or a formulation amount of

the present compound, polyethylene glycol, an additive and/or a solvent in the above-mentioned preparation examples 1 to 5.

1. Stability evaluation test (1)

Stability of the pharmaceutical composition of the present invention was investigated.

1-1. Preparation of formulation to be tested

A suitable amount of polyethylene glycol 400 (Nacalai Tesque Inc.) was added to 0.10 g of the compound (2-[[[2-[(hydroxyacetyl)amino]-4-pyridinyl]methyl]thio]-N-[4-(trifluoromethoxy)phenyl]-3-pyridinecaboxamide, in the following, it is also called as Compound A; prepared according to the method described in US Patent Application Publication No. 2007/0149574A) represented by the above-mentioned formula (2), and the whole amount was made 10 mL and dissolved under stirring to prepare a formulation of Example 1.

In the same manner as in the preparation method of Example 1, formulations of Examples 2 to 4 shown in Table 1 were prepared. The polyethylene glycols used were available from Nacalai Tesque Inc.

1-2. Test method

In 3 mL of a glass vial (Wheaton) was filled 0.4 mL of the formulation to be tested, and the amount of 2-[[[2-[(hydroxyacetyl)amino]-4-pyridinyl]methyl]thio]-N-[4-(trifluoromethoxy)phenyl]-3-pyridinecaboxamide when it was preserved at 40°C and 60°C until each 3 months and 4 weeks were determined by using high-performance liquid chromatography (HPLC) to calculate the residual ratio (%) thereof.

1-3. Test results and consideration

The test results are shown in Table 1.

[Table 1]

| | | | Example 1 | Example 2 | Example 3 | Example 4 |
|---|---|---|---|---|---|---|
| Compound A | | | 0.1g | 0.1g | 0.1g | 0.1g |
| Polyethylene glycol 400 | | | Quantum sufficit | - | - | - |
| Polyethylene glycol 200 | | | - | Quantum sufficit | - | - |
| Polyethylene glycol 300 | | | - | - | Quantum sufficit | - |
| Polyethylene glycol 600 | | | - | - | - | Quantum sufficit |
| Whole amount | | | 10mL | 10mL | 10mL | 10mL |
| Residual ratio (%) | 60°C | 2 weeks | 98.7 | 96.7 | 98.2 | 100.3 |
| | | 4 weeks | 93.2 | 85.8 | 90.2 | 96.5 |
| | 40°C | 1 month | 98.4 | 97.8 | 98.8 | 99.1 |
| | | 2 months | 99.6 | 96.9 | 98.5 | 99.3 |
| | | 3 months | 100.2 | 96.2 | 95.9 | 97.6 |

As can be clearly seen from Table 1, formulations of Examples 1 to 4 maintained high residual ratio at 40°C for 3 months and at 60°C for 4 weeks. In particular, high residual ratios were shown when polyethylene glycol 400 or polyethylene glycol 600 had been used. From the above, it was confirmed that the compositions of the present invention had excellent stability.

2. Stability evaluation test (2)

Stability of the pharmaceutical composition of the present invention was investigated.

2-1. Preparation of formulation to be tested

Suitable amount of polyethylene glycol 400 (NOF CORPORATION) was added to 0.6 g or 12 g of Compound A and the whole amount was made 240 mL, and the mixture was dissolved by stirring to prepare formulations of Examples 5 and 6.

2-2. Test method

In 3 mL of glass vial (Wheaton) was filled 2 mL of the formulation to be tested, and the amount of 2-[[[2-[(hydroxyacetyl)amino]-4-pyridinyl]methyl]thio]-N-[4-(trifluoromethoxy)phenyl]-3-pyridinecaboxamide when it was preserved at 25°C until each 3 months was determined by using high-performance liquid chromatography (HPLC) to calculate the residual ratio (%) thereof.

2-3. Test results and consideration

The test results are shown in Table 2.

[Table 2]

| | | Example 5 | Example 6 |
|---|---|---|---|
| Compound A | | 0.6g | 12g |
| Polyethylene glycol 400 | | Quantum sufficit | Quantum sufficit |
| Whole amount | | 240mL | 240mL |
| Residual ratio (%) | 25°C 3 months | 93.7 | 98.3 |

As can be clearly seen from Table 2, the formulations of Example 5 and 6 maintained high residual ratios at 25°C for 3 months. From the above, it was confirmed that the compositions of the present invention had excellent stability.

3. Stability evaluation test (3)

Stabilities of the compositions of the present invention containing dimethylsulfoxide and a nonionic surfactant were investigated.

3-1. Preparation of formulation to be tested

Example 7 shown in Table 3 was prepared by adding 1.50 g of dimethylsulfoxide and 32.11 g of polyethylene glycol 400 to 0.75 g of Compound A, and dissolved by stirring. Also, in the same manner as in the preparation method of Example 1, formulations of Examples 8 and 9 shown in Table 3 were prepared. Polyethylene glycol 400 used was available from NOF CORPORATION, dimethylsulfoxide from Nacalai Tesque Inc., polysorbate 20 from Nikko Chemicals Co., Ltd., and Polyoxyl 35 castor oil from BASF.

3-2. Test method

In 2 mL glass vial (Shiotani Glass Co., Ltd.) was filled 0.4 mL of the formulation to be tested, and the amount of 2-[[[2-[(hydroxyacetyl)amino]-4-pyridinyl]-methyl]thio]-N-[4-(trifluoromethoxy)phenyl]-3-pyridinecaboxamide when it was preserved at 60°C until 4 weeks were determined by using high-performance liquid chromatography (HPLC) to calculate the residual ratio (%) thereof.

3-3. Test results and consideration

The test results are shown in Table 3.

[Table 3]

| | Example 7 | Example 8 | Example 9 |
|---|---|---|---|
| Compound A | 0.75g | 0.38g | 0.37g |

(continued)

| | | Example 7 | Example 8 | Example 9 |
|---|---|---|---|---|
| Dimethylsulfoxide | | 1.50g | - | - |
| Polysorbate 20 | | - | 0.75g | - |
| Polyoxyl 35 castor oil | | - | - | 0.75g |
| Polyethylene glycol 400 | | 32.11g | Quantum sufficit | Quantum sufficit |
| Whole amount | | About 30mL | 15mL | 15mL |
| Residual ratio (%) | 60°C, 4 weeks | 84.7 | 75.9 | 75.3 |

As can be clearly seen from Table 3, the formulations of Examples 7 to 9 maintained high residual ratios at 60°C for 4 weeks. From the above, it was confirmed that the compositions of the present invention had excellent stability even when dimethylsulfoxide or a nonionic surfactant had been added.

4. Pharmacokinetics test

Pharmacokinetics after intravitreal administration of the compositions of the present invention were investigated.

4-1. Preparation of formulation to be tested

To 100.5 mg of Compound A was added 1.8 mL of polyethylene glycol 400 (Nacalai Tesque Inc.), the mixture was stirred, and after confirmation of dissolution, a suitable amount of polyethylene glycol 400 was added to the mixture to make the whole amount 2.0 mL to prepare a formulation of Example 10.

To 50.4 mg of Compound A was added 1.8 mL of polyethylene glycol 400 (Nacalai Tesque Inc.), the mixture was stirred, and after confirmation of dissolution, a suitable amount of polyethylene glycol 400 was added to the mixture to make the whole amount 2.0 mL to prepare a formulation of Example 11.

About 400 mL of water for injection (Otsuka Pharmaceutical Co., Ltd.) was added to 2.5 g of conc. glycerin (Kao Corporation), 1.0 g of polysorbate 80 (Sanyo Chemical Industries, Ltd.), 12 g of sodium hydrogen phosphate hydrate (Taihei Chemical Industrial Co., Ltd.), 1.2 g of sodium dihydrogen phosphate (Taihei Chemical Industrial Co., Ltd.) and 0.005 g of hypromellose (Shin-Etsu Chemical Co., Ltd.), and the mixture was dissolved by stirring. Water for injection was further added to make the whole amount 500 mL and this was made a base material for suspension. To 75.3 mg of the present compound A was added 5.0 mL of the base material for suspension, and the mixture was stirred to suspend therein to prepare a formulation of Comparative example 1.

4-2. Test method

The formulations to be tested were each injected into the vitreous body of the eyes of Japanese white rabbits. The solution injected at this time formed a lump to the surrounding medium. After 4, 8, 12 or 28 weeks from the injection, the rabbits were euthanized according to the standard procedure.

After enucleating eyeballs, the vitreous bodies were separated, and charged in a 50 mL tube (the tare weight has already been known) into which two zirconia beads (5 mm) had been charged. After measuring the weight of the tube, the tissue was homogenized (1,100 rpm, 10 minutes) by Shake Master Auto by charging methanol therein. After centrifugal separation (3,000 rpm, room temperature, 10 minutes), the supernatant was made a vitreous body matrix sample. The prepared vitreous body matrix sample was preserved by freezing (set temperature: -80°C) in an ultracold freezing storehouse until the use.

After collecting the vitreous body, the retinal surface was washed with a saline. The retina and choroid at around an optic disk was collected, and charged in a 2 mL tube (the tare weight has already been known) into which 0.5 mL of 2% formic acid containing 10 mg/mL of sodium fluoride and two zirconia beads (3 mm) had been charged therein. The weight of the tube into which the retina and choroid has been charged therein was measured. After homogenizing (1,100 rpm, 10 minutes) by Shake Master Auto, 0.5 mL of methanol was added thereto and the mixture was stirred. The mixture was centrifuged (11,000 rpm, 4°C, 10 minutes), and the supernatant was made a retinochoroidal matrix sample. It was preserved by freezing (set temperature: -80°C) in an ultracold freezing storehouse until the use.

Contents of Compound A in the vitreous body and in the retinochoroidal tissue subjected to decomposition preventive treatment by sodium fluoride were determined by high-performance liquid chromatography/tandem type mass spectrometer (HPLC/MS/MS) using an internal standard. The lump observed in the vitreous body was analyzed in the

state contained in the vitreous body portion.

Incidentally, at each time point, residual ratios or concentrations of Compound A obtained from the eyes of the respective rabbits were each added, and divided by the whole number of the eyes analyzed to calculate an average residual ratio or concentration of Compound A. In this experiment, at each time point, an average of eyes of two rabbits (3 or 4 eyes at each time point) at each time point was shown at the each time point.

The residual ratio of Compound A in the vitreous body was calculated by, after measurement of the concentration of Compound A, the vitreous body weight was multiplied to the concentration value, and dividing it with the administration amount of Compound A. This measured value shows the residual amount of Compound A in the vitreous body containing the lump at the tissue collecting time after the administration.

The concentration in the retina and the choroidea was calculated by calculating the amount of Compound A measured, then, dividing it by the amount of the retina and choroid used for the analysis. This measured value shows the concentration of Compound A transferred from the vitreous body tissue to the retina and the choroidea.

4-3. Test results and consideration

The residual ratio (%) of Compound A existing in the vitreous body after 4 weeks from the injection, 8 weeks, 12 weeks and 28 weeks, and the concentration (μg/g) of Compound A in the retina and choroid after 4 weeks from the injection were shown in Table 4.

[Table 4]

| | | Example 10 | Example 11 | Comparative example 1 |
|---|---|---|---|---|
| Compound A | | 100.5mg | 50.4mg | 7.53g (calculated value) |
| Polyethylene glycol 400 | | Quantum sufficit | Quantum sufficit | - |
| Conc. glycerin | | - | - | 2.5g |
| Sodium hydrogen phosphate hydrate | | - | - | 12g |
| Sodium dihydrogen phosphate | | - | - | 1.2g |
| Polysorbate 80 | | - | - | 1.0g |
| Hypromellose | | - | - | 0.005g |
| Water for injection | | - | - | Quantum sufficit |
| Whole amount | | 2.0mL | 2.0mL | 500mL |
| Administered amount to vitreous body | | 20μL (1.0mg/eye) | 20μL (0.5mg/eye) | 50μL (0.75mg/eye) |
| Compound A residual ratio (%) in vitreous body | 4 weeks | 73.3 | 71.9 | 15.8 |
| | 8 weeks | 54.9 | 54.1 | - |
| | 12 weeks | 56.0 | - | 8.8 |
| | 28 weeks | - | 22.2 | - |
| Compound A concentration (μg/g) in retinochoroidal | 4 weeks | 1.45 | 0.716 | 0.102 |

As can be clearly seen from Table 4, the formulations of Example 10 and 11 showed sufficiently high residual ratio in the vitreous body and the retinochoroidal concentration as compared with those of the formulation of Comparative example 1. From the above, it was confirmed that the compositions of the present invention has excellent sustained releasability when it was administered into the vitreous body.

5. Pharmacological test

By using the VEGF induced rabbit retinal vascular permeability promotion model, usefulness of the polyethylene glycol 400 formulation of Compound A was evaluated. Incidentally, the VEGF has been reported to promote retinochoroidal vascular permeability by intravitreal administration (Invest Ophthalmol Vis Sci. 2013; 54(1):503-11.), and has generally been used for preparation of a pathological condition accompanied by retinal and choroidal vascular disorder (for example, diabetic retinopathy, diabetic macular edema, retinal vein occlusion, retinal artery occlusion, exudative age-related macular degeneration, etc.).

5-1. Preparation of formulation to be tested

To 1.5 g of the present compound A was added 12 mL of polyethylene glycol 400 (NOF CORPORATION) and the mixture was stirred, and after confirming the dissolution thereof, a suitable amount of polyethylene glycol 400 was added to make the whole amount 15 mL.

5-2. Test method

(Administration method of medicine)

Rabbits were intramuscularly administered 1 mL/kg of a mixed solution (7:1) of a 5% ketamine injection solution and a 2% xylazine injection solution to carry out general anesthesia, and dropped eye drops containing 0.5% tropicamide and 0.5% phenylephrine hydrochloride to make the eyes mydriasis. Thereafter, 5 μL of Compound A solution (=0.5 mg/eye) was injected into the vitreous body using a 27G needle so that the crystalline lens and the retina were not injured. Incidentally, to the rabbits of the base material administered group, polyethylene glycol 400 was administered in the same manner.

(Preparation method of VEGF induced rabbit retinal vascular permeability promotion model)

After 2 months from administration of the medicine, rabbits were intramuscularly administered 1 mL/kg of a mixed solution (7:1) of a 5% ketamine injection solution and a 2% xylazine injection solution to carry out general anesthesia, and dropped eye drops containing 0.5% tropicamide and 0.5% phenylephrine hydrochloride to make the eyes mydriasis. Thereafter, 10 μL of VEGF (50 μg/mL) was injected into the vitreous body using a 27G needle so that the crystalline lens and the retina were not injured. The rabbits of the normal group were similarly administered PBS (phosphate buffer solution) in place of VEGF in the same manner.

(Evaluation method)

After 2 days from the VEGF administration, 0.1 mL/kg of a 10% fluorescein solution was intravenously administered to rabbits. After 2 hours from administration of the fluorescein, the fluorescein concentration in the vitreous body was measured by fluorophotometry, and this was used as an index of the retinal vascular permeability.

Thereafter, according to Formula 1, an inhibition ratio (%) of the administered medicine to the retinal vascular permeability promotion caused by VEGF was calculated. The results are shown in Table 5. Incidentally, a number of examples in each group was 8, and an average value thereof was used for calculating the inhibition ratio.

[0108] [Formula 1] Retinal vascular permeability inhibition ratio (%)=$(A_Y-A_Z)/(A_Y-A_X)\times100$

$A_X$: Fluorescein concentration in vitreous body of base material (=polyethylene glycol 400) administered+PBS administered group

$A_Y$: Fluorescein concentration in vitreous body of base material (=polyethylene glycol 400) administered+VEGF administered group

Az: Fluorescein concentration in vitreous body of Compound A administered+VEGF administered group

[Table 5]

| Substance to be tested | Inhibition ratio (%) |
|---|---|
| Compound A 0.5mg/eye | 95.3 |

**[0104]** From the results mentioned above, it could be confirmed that the polyethylene glycol 400 formulation of Compound A had an excellent inhibition effect against the VEGF induced retinal vascular permeability promotion even after two months from the intravitreal administration. It has been shown that the composition of the present invention had remarkable effects continuously for a long period of time to the posterior ocular diseases to which retinal and choroidal vascular disorder pertains such as diabetic retinopathy, diabetic macular edema, retinal vein occlusion, retinal artery occlusion, exudative age-related macular degeneration, etc.

## Claims

1. A pharmaceutical composition which comprises a compound represented by the formula (1):

[Formula 3]

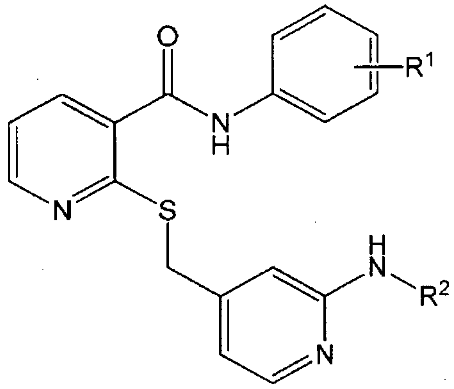

(1)

wherein

$R^1$ represents a hydrogen atom, a halogen atom, a hydroxyl group, a $C_{1-6}$ alkyl group, a $C_{1-6}$ alkyl group substituted by one or more halogen atoms, a $C_{1-6}$ alkoxyl group or a $C_{1-6}$ alkoxyl group substituted by one or more halogen atoms; and
$R^2$ represents a hydrogen atom, a $C_{1-6}$ alkyl group, a $C_{1-6}$ alkylcarbonyl group or a $C_{1-6}$ alkylcarbonyl group substituted by one or more hydroxyl groups,

or a salt thereof and polyethylene glycol.

2. The pharmaceutical composition according to claim 1, wherein

$R^1$ represents a $C_{1-6}$ alkoxyl group or a $C_{1-6}$ alkoxyl group substituted by one or more halogen atoms; and
$R^2$ represents a $C_{1-6}$ alkylcarbonyl group or a $C_{1-6}$ alkylcarbonyl group substituted by one or more hydroxyl groups in the formula (1).

3. The pharmaceutical composition according to claim 1, wherein

$R^1$ represents a $C_{1-6}$ alkoxyl group substituted by one or more halogen atoms; and
$R^2$ represents a $C_{1-6}$ alkylcarbonyl group substituted by one or more hydroxyl groups in the formula (1).

4. The pharmaceutical composition according to claim 1, wherein the compound represented by the formula (1) is 2-[[[2-[(hydroxyacetyl)amino]-4-pyridinyl]methyl] - thio]-N-[4-(trifluoromethoxy)phenyl]-3-pyridinecaboxamide.

**5.** The pharmaceutical composition according to any one of claims 1 to 4, wherein an average molecular weight of the polyethylene glycol is within the range of 100 to 2,000.

**6.** The pharmaceutical composition according to any one of claims 1 to 4, wherein an average molecular weight of the polyethylene glycol is within the range of 200 to 600.

**7.** The pharmaceutical composition according to any one of claims 1 to 4, wherein the polyethylene glycol is PEG 400.

**8.** The pharmaceutical composition according to any one of claims 1 to 7, wherein the amount of the polyethylene glycol in the pharmaceutical composition is 70 to 99.99% (w/w).

**9.** The pharmaceutical composition according to any one of claims 1 to 8, wherein the amount of the compound represented by the formula (1) or a salt thereof is 0.01 to 20% (w/v).

**10.** The pharmaceutical composition according to any one of claims 1 to 9 for prophylaxis or treatment of an eye disease.

**11.** The pharmaceutical composition according to claim 10, wherein the eye disease is age-related macular degeneration, diabetic retinopathy, retinopathy of prematurity, retinal vein occlusion, retinal artery occlusion, polypoidal choroidal vasculopathy, retinal angiomatous proliferation, myopic choroidal neovascularization, diabetic macular edema, ocular tumor, radiation retinopathy, iris rubeosis, neovascular glaucoma or proliferative vitreoretinopathy (PVR).

**12.** The pharmaceutical composition according to claim 10 or 11, which is for intravitreal administration.

**13.** The pharmaceutical composition according to claim 12, which is administered 1 to 100 μL per once.

**14.** The pharmaceutical composition according to claim 12 or 13, which is administered with an interval of once per week to once per 3 years.

**15.** A method for stabilizing a compound represented by the formula (1) or a salt thereof which comprises dissolving the compound represented by the formula (1) or a salt thereof in polyethylene glycol.

**16.** The method according to claim 15, wherein the compound represented by the formula (1) is 2-[[[2-[(hydroxyacetyl)amino]-4-pyridinyl]methyl]thio]-N-[4-(trifluoromethoxy)phenyl]-3-pyridinecaboxamide.

## INTERNATIONAL SEARCH REPORT

International application No.
PCT/JP2014/074698

A. CLASSIFICATION OF SUBJECT MATTER
*A61K31/444*(2006.01)i, *A61K9/08*(2006.01)i, *A61K47/34*(2006.01)i, *A61P3/10*(2006.01)i, *A61P27/02*(2006.01)i, *A61P27/06*(2006.01)i, *A61P35/00*(2006.01)i

According to International Patent Classification (IPC) or to both national classification and IPC

B. FIELDS SEARCHED

Minimum documentation searched (classification system followed by classification symbols)
A61K31/444, A61K9/08, A61K47/34, A61P3/10, A61P27/02, A61P27/06, A61P35/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/REGISTRY/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2006-96739 A (Santen Pharmaceutical Co., Ltd.), 13 April 2006 (13.04.2006), claims; paragraphs [0001], [0118], [0125]; table 1; Pharmacological Tests & US 2007/0149574 A1 & EP 1717229 A1 & WO 2005/085201 A1 & KR 10-2006-0135818 A & CN 1918127 A | 1-16<br>1-16 |
| Y | JP 59-101478 A (Sunstar Inc.), 12 June 1984 (12.06.1984), claims; page 1, right column, line 12 to page 2, upper left column, line 8; experimental examples; examples (Family: none) | 1-16 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>30 October, 2014 (30.10.14) | Date of mailing of the international search report<br>11 November, 2014 (11.11.14) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No. PCT/JP2014/074698

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2013-518130 A (Eagle Pharmaceuticals, Inc.), 20 May 2013 (20.05.2013), claims; paragraph [0004]; examples & US 2011/0184036 A1 & EP 2528602 A1 & WO 2011/094565 A1 | 1-16 |
| Y | JP 58-219108 A (Beecham Group PLC), 20 December 1983 (20.12.1983), claims; page 2, upper left column, lines 1 to 10; examples & US 4524075 A1 & EP 95897 A2 | 1-16 |
| Y | JP 9-110698 A (Taiho Pharmaceutical Co., Ltd.), 28 April 1997 (28.04.1997), claims; paragraph [0001]; examples (Family: none) | 1-16 |
| Y | JP 2011-504934 A (Merial Ltd.), 17 February 2011 (17.02.2011), claims; paragraph [0019]; examples & US 2009/0163575 A1 & EP 2222168 A1 & WO 2009/070687 A1 | 1-16 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20070149574 A
- US 20120116088 A
- WO 2007076358 A
- WO 2009014510 A
- WO 2010101971 A
- US 8367097 B


### Non-patent literature cited in the description


- *Invest Ophthalmol Vis Sci.,* 2013, vol. 54 (1), 503-11